# EUROPÄISCHE PATENTANMELDUNG

(11) **EP 0 761 234 A2**
(43) Veröffentlichungstag der Anmeldung: **12.03.1997**
(21) Anmeldenummer: 96113676.9
(22) Anmeldetag: 27.08.1996
(51) Int. Cl.: A61K 47/02, A61K 9/20, A61K 31/19

(54) **Pharmazeutisches Kombinationspräparat mit Ketoprofen**

(30) Priorität: 08.09.1995 DE 19533162
(71) Anmelder: BAYER AG, 51368 Leverkusen (DE)
(72) Erfinder: Maasz, Joachim, Dr., Convent Station, NJ 07960 (US); Hürner, Ingrid, 51375 Leverkusen (US); Kurka, Peter, Dr., 40764 Langenfeld (DE); Lange, Ralph, Dr., 42489 Wülfrath (DE)

(57) **Zusammenfassung**

Die Erfindung betrifft die kombinierte Anwendung von Ketoprofen mit speziellen anorganischen, basischen Substanzen mit verbesserter Wirkungsqualität.

## Beschreibung

Die Erfindung betrifft die kombinierte Anwendung von Ketoprofen mit speziellen anorganischen, basischen Substanzen mit verbesserter Wirkungsqualität.

Es ist bereits bekannt, daß basische Stoffe wie Magnesiumhydroxid, Magnesiumoxid und Natriumhydrogencarbonat oder deren Mischungen Einfluß auf die Absorption bestimmter Wirkstoffe wie Anthranilsäurederivate, Propionsäurederivate, Essigsäurederivate, Salicylsäurederivate oder deren Salze oder von Pyrazololen oder Benzothiazinderivaten besitzen (vgl. Neuvonen WO 89/07439). In dieser Anmeldung wird als Beispiel eines Propionsäurederivates auch Ketoprofen erwähnt.

Für den Fachmann ist es auch bekannt, daß der absorptionsregulierende Effekt bestimmter Zusätze nicht für alle Wirkstoffe verallgemeinert werden kann (vgl. D'Arcy et al., Drug Intelligence and Clinical Pharmacy, 21, 607 (1987)). Die erteilten Ansprüche der obengenannten PCT-Anmeldung von Neuvonen wurden auf die speziellen Wirkstoffe Tolfenaminsäure, Mefenaminsäure und Ibuprofen beschränkt und als basische Kombinationspartner werden nur noch Magnesiumhydroxid und Magnesiumoxid beansprucht. Dies bestätigt die Aussage der Publikation von D'Arcy et al., daß die Wirkung von basischen Stoffen wie Antacida auf das Resorptionsverhalten von Wirkstoffen nicht vorhergesehen werden kann. Auch die spätere Publikation von Neuvonen in Br. J. Clin. Pharmac. 31, 263 (1991) zeigt anhand von zwei Cross-Over-Studien, daß durch Zugabe von Magnesiumhydroxid nur im Fall von Ibuprofen eine höhere Plasmakonzentration auftritt. Für den Fall des Ketoprofens wurde weder eine signifikante Zunahme der Absorptionsgeschwindigkeit noch ein erhöhtes Ausmaß der Absorption gefunden.

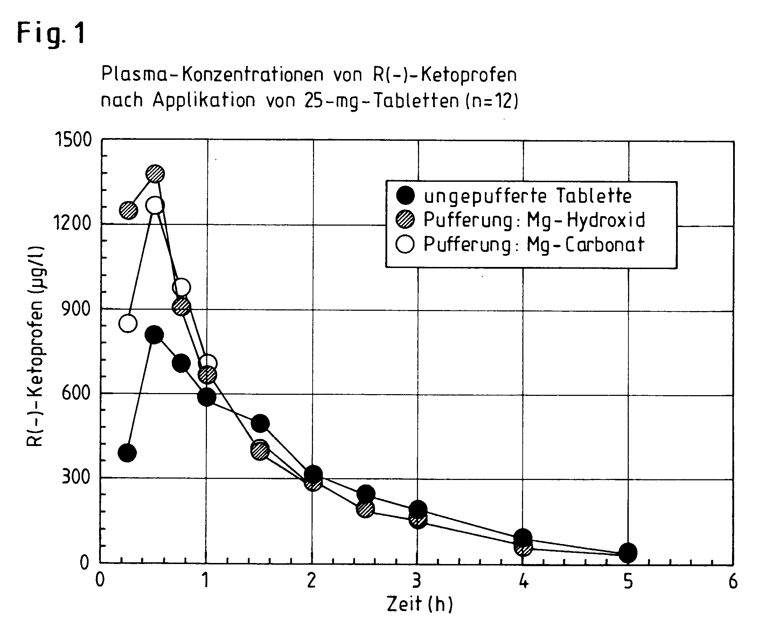

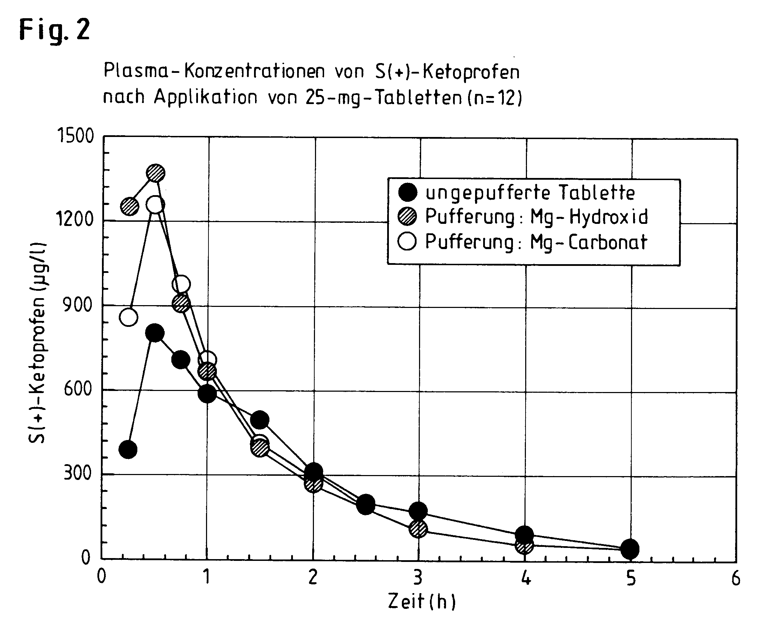

Bei Kenntnis dieses Standes der Technik konnte nicht erwartet werden, daß durch eine Kombination von Ketoprofen in racemischer Form und in Form ihrer S(+)- und R(-)-Enantiomere mit basischen Hilfsstoffen wie Magnesiumhydroxid und Magnesiumcarbonat im Vergleich zu nicht gepufferten Tabletten eine schnellere Wirkung und eine signifikante Erhöhung des maximalen Plasmaspiegels erreicht wird, wie aus den Abbildungen 1 und 2 ersichtlich ist.

Die absorptionsbeschleunigende Wirkung von Magnesiumhydroxid und Magnesiumcarbonat ist aus dem Verhältnis C_{max/}AUC gemäß Tabelle 1 ersichtlich.

Weiterhin beginnt der Bereich der tₘₐₓ-Werte im Fall der gepufferten Tabletten gemäß erfindungsgemäßer Kombination früher und zeigt eine geringere Streuung als im Fall der nicht gepufferten Tabletten. Die vergleichenden Untersuchungen zeigen ebenfalls, daß die interindividuelle Variante der individuellen Plasmaspiegel im Fall der gepufferten erfindungsgemäßen Kombinationstabletten kleiner ist als im Fall der nicht gepufferten Tabletten.

Die gefundenen Unterschiede zu den maximalen Plasmakonzentrationen (Cₘₐₓ) der drei unterschiedlichen Tablettenformulierungen sind signifikant. Besonders die Magnesiumhydroxid- gepufferte Ketoprofentablette führt dazu, daß früher als bei der nicht gepufferten Tablette höhere Plasmakonzentrationen des Ketoprofens erreicht werden. Insbesondere in der Schmerzindikation ist es daher empfehlenswert die gepufferten Tabletten mit ihrem rascheren Wirkungseintritt im Vergleich zu den nicht gepufferten Tabletten anzuwenden.

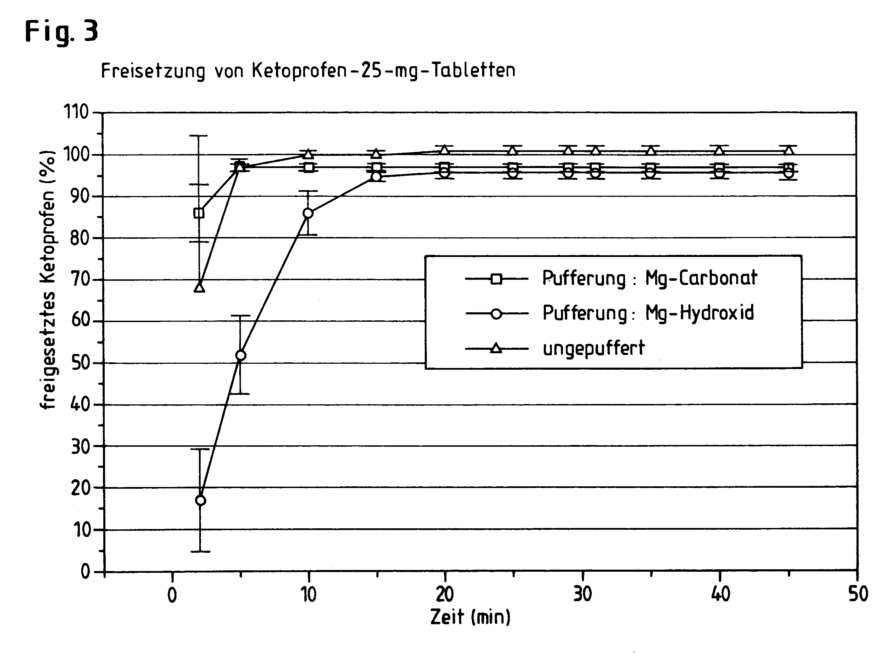

Die erfinderische Tätigkeit liegt nicht nur in der Überwindung des literaturbekannten Vorurteils, daß die Absorption von Ketoprofen durch basische Stoffe nicht positiv beeinflußt werden kann, sondern wird noch durch eigene in vitro-Test verstärkt. Eine Untersuchung der Freisetzung von Ketoprofen-Tabletten (25 mg) in-vitro ergibt, daß die Formulierung mit der Kombination Ketoprofen und Magnesiumhydroxid am langsamsten freisetzt, wie aus Abbildung 3 ersichtlich ist. Bei diesen negativen in-vitro-Ergebnissen konnte nicht erwartet werden, daß insbesondere die Kombination Ketoprofen + Magnesiumhydroxid in-vivo ein so vorteilhaftes Absorptionsverhalten zeigt.

Von besonderem Interesse sind erfindungsgemäße Fixkombinationen in Form von Tabletten, Brausetabletten, Kapseln, Granulaten, Pulvermischungen, Suspensionen, Emulsionen und Tropfen, die vorzugsweise 1 Gew.-Teil Ketoprofenracemat oder S(+)-Ketoprofen oder R(-)-Ketoprofen in reiner Form oder als Mischungen im Gewichtsverhältnis von 1 zu 99 bis 99 zu 1 enthalten und 1 bis 25 Gew.-Teile des basischen puffernden Zusatzes, insbesondere Magnesiumhydroxid, enthalten.

Die Pufferkapazität der basischen Kombinationspartner in den erfindungsgemäßen Darreichungsformen beträgt vorzugsweise mindestens 3 Milliequivalent (meq).

Bevorzugt sind orale Darreichungsformen mit geringer individueller Streuung der Plasmakonzentrationen, einer erhöhten Absorptionsgeschwindigkeit und höheren maximalen Plasmakonzentration.

Die erfindungsgemäßen Fixkombinationen werden nach üblichen Methoden, z.B. durch Mischen und anschließendes Verpressen oder durch Auflösen der einzelnen Komponenten hergestellt.

### Ausführungsbeispiele

### Beispiel 1

Die Substanzen des Beispiels 1 werden zu einer Tablette verarbeitet, die Ketoprofen in vitro mit mäßiger Geschwindigkeit freisetzt.

| unlackierte Tablette | |
|---|---|
| Ketoprofen (Racemat) | 25,0 mg |
| Magnesiumhydroxid | 150,4 mg |
| kolloidale Kieselsäure | 12,0 mg |
| Natrium-Carboxymethylstärke | 7,0 mg |
| Natrium-Citrat, tertiär | 50,0 mg |
| Magnesiumstearat | 0,6 mg |

| Lackhülle | |
|---|---|
| HPM Cellulose | 1,2 mg |
| Polyethylenglykol 4000 | 0,4 mg |
| Titandioxid | 0,4 mg |
| Gesamtgewicht | 147,0 mg |

### Herstellung

Ketoprofen, Magnesiumhydroxid, Natrium-Carboxymethylstärke und Natrium-Citrat, werden wäßrig granuliert und anschließend getrocknet.

Zu diesem Granulat werden die restlichen Bestandteile zugemischt (kolloidale Kieselsäure, Magnesiumstearat) und diese Mischung auf geeigneten Tablettenpressen zu Tabletten mit einem Durchmesser von 8 mm verpreßt.

### Beispiel 1a und b

In analoger Weise werden Tabletten mit S(+)- und R(-)-Ketoprofen hergestellt.

### Beispiel 2

Die Substanzen des Beispiels 2 werden zu einer Tablette verarbeitet, die Ketoprofen in vitro rasch freisetzt.

| unlackierte Tablette | |
|---|---|
| Ketoprofen (Racemat) | 25,0 mg |
| Magnesiumcarbonat, basisch | 258,0 mg |
| Natrium-Carboxymethylstärke | 10,0 mg |
| Polyvinylpyrrolidon 25 | 7,4 mg |
| kolloidale Kieselsäure | 2,0 mg |
| Magnesiumstearat | 0,6 mg |

| Lackhülle | |
|---|---|
| HPM Cellulose | 1,8 mg |
| Polyethylenglykol 4000 | 0,6 mg |
| Titandioxid | 0,6 mg |
| Gesamtgewicht | 306,0 mg |

### Herstellung

Ketoprofen, Magnesiumcarbonat, Natrium-Carboxymethylstärke und PVP werden wäßrig granuliert und anschließend getrocknet.

Zu diesem Granulat werden die restlichen Bestandteile zugemischt (kolloidale Kieselsäure, Magnesiumstearat) und diese Mischung auf geeigneten Tablettenpressen zu Tabletten mit einem Durchmesser von 9 mm verpreßt.

### Vergleichsbeispiel 3 (ohne Puffer)

Die Substanzen des Beispiels 3 werden zu einer Tablette verarbeitet, die Ketoprofen in vitro rasch freisetzt. Die Tablette enthält keinen puffernden Zusatz.

| | |
|---|---|
| Ketoprofen | 25,0 mg |
| Maisstärke | 48,0 mg |
| Avicel | 30,0 mg |
| Milchzucker | 32,0 mg |
| Natrium-Carboxymethylcellulose (Ac-Di-Sol) | 4,3 mg |
| Magnesiumstearat | 0,7 mg |

| Lackhülle | |
|---|---|
| HPM Cellulose | 0,6 mg |
| Polyethylenglykol 4000 | 0,2 mg |
| Titandioxid | 0,2 mg |
| Gesamtgewicht | 141,0 mg |

### Herstellung

Ketoprofen, Maisstärke, Avicel, Milchzucker und Ac-Di-Sol werden wäßrig granuliert und anschließend getrocknet.

Dieses Granulat wird mit Magnesiumstearat gemischt und auf geeigneten Tablettenpressen zu Tabletten mit einem Durchmesser von 7 mm verpreßt.

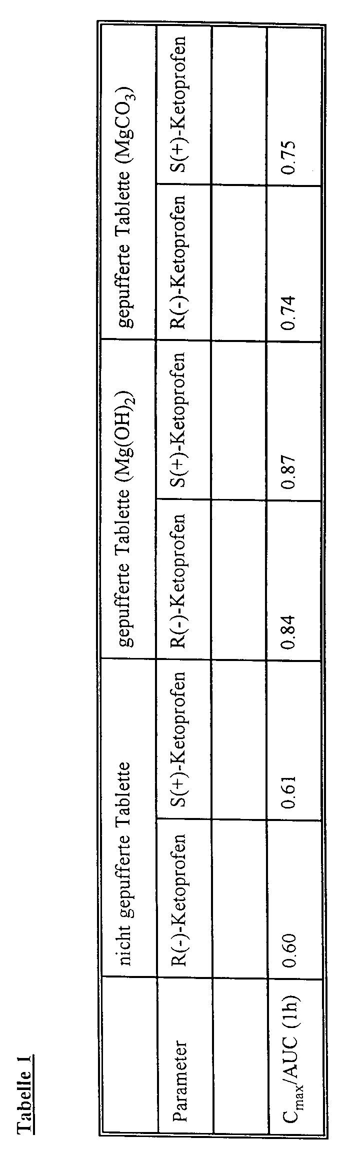

## Patentansprüche

1. Ketoprofenzubereitung mit verbesserter Wirkungsqualität enthaltend eine Kombination von 1 Gew.-Teil Ketoprofen und 1 bis 25 Gew.-Teilen einer anorganischen basischen Puffersubstanz.

2. Kombinationspräparat gemäß Anspruch 1, dadurch gekennzeichnet, daß sie als anorganisch basische Puffersubstanz Magnesiumhydroxid, Magnesiumoxid oder Magnesiumcarbonat enthalten.

3. Kombinationspräparat gemäß Anspruch 1, dadurch gekennzeichnet, daß der Wirkstoff Ketoprofen in Form seiner Enantiomeren S(+)- oder R(-)-Ketoprofen in reiner Form oder als Mischungen im Verhältnis 1:99 bis 99:1 enthält.

4. Kombinationspräparat gemäß Anspruch 1, dadurch gekennzeichnet, daß es Ketoprofen und Magnesiumhydroxid enthält.

5. Kombinationspräparat gemäß Anspruch 1 in Form von Tabletten, Kapseln, Granulaten, Pulvermischungen oder Suspensionen.

6. Verfahren zur Herstellung von Kombinationszubereitungen gemäß Anspruch 1, dadurch gekennzeichnet, daß man 1 Gew.-Teil Ketoprofen mit 1 bis 25 Gew.-Teilen der anorganischen basischen Substanzen vermischt und in eine geeignete Applikationsform überführt.
